# EUROPEAN PATENT APPLICATION

(11) **EP 1 405 665 A1**
(43) Date of publication of application: **07.04.2004**
(21) Application number: 02022037.2
(22) Date of filing: 01.10.2002
(51) Int. Cl.: B01J 13/04

(54) **Particle embedded with chemical substances and method of producing a particle**

(71) Applicant: Alarvita Biolife Corporation, Taipei (TW)
(72) Inventor: Tsou, Te Chun, Jungli City, Taoyuan, Taiwan 320 (TW); Kong, Zwe-Ling, Keelung, Taiwan (TW); Chang, Ke Liang, Shrlin Chiu, Taipei, Taiwan (TW); Lee, Shing-Mou, Bali Shiang, Taipei, Taiwan (TW)
(74) Representative: Weber, Joachim, Dr.

(57) **Abstract**

A particle embedded with chemical substances, especially with physiological activity substances, is disclosed. The particle has (a) the embedded substances, embedded in base materials and ranged from about 0.1 to 70 % by weight based on the total weight of the particle; and (b) the base material, selected from hydrogenated plant oil, hydrogenated animal oil, C8-C64 saturated fatty acid or the derivatives thereof, and ranged from about 30 to 99.9 % by weight based on the total weight of the particle. Also, the present invention discloses a method for producing the particle.

## Description

### BACKGROUND OF THE INVENTION

### Field of the Invention

The present invention relates to a particle with embedded chemical substances, and especially relates to a particle with embedded physiologically active substances. Also, the present invention relates to a method of producing a particle with embedded chemical substances.

### Description of the Related Art

Based on the present state of science and technology, the techniques of producing a particle from oil are mainly separated into two types. One is coacervation, and the other uses an atomized-flow restrictor. Coacervation forms uniform oil-water composition from the oily substance and water by mixing, shaking and emulsifying.

U.S. Pat. No. 6,153,657 discloses a process of producing particles in which (a) water is mixed with an oily substance, a high molecular weight surfactant and a low molecular weight surfactant, the mixture are stirred at 40°C or less, to provide an O/W type emulsion. The stirring step could be performed by a homo-mixer rotating at the high speed, such as 3,000 to 15,000 rpm. (b) The resultant oil-in-water (O/W) emulsion is heated to a temperature at which the O/W emulsion is gelled while stirring is performed by, for example, a homo-mixer at 3,000 to 15,000 rpm. While stirring the O/W emulsion, the resultant O/W emulsion gel is further heated to a temperature at which the O/W emulsion gel is phase inverted to a water-in-oil (W/O) emulsion gel. (c) The resultant W/O emulsion gel is then cooled to a temperature below the gelling temperature achieved in the previous step to convert the W/O emulsion gel to an O/W emulsion. Then, the resultant product is stirred at a temperature of 40°C or less to provide an O/W emulsion containing no solvent and stable when diluted with water. However, the defects of this conventional method are the difficulty of controlling the operating conditions and the complexity of executing the steps. Furthermore, it takes too much time to produce the particles.

The other conventional process of producing particles uses an atomized-flow restrictor. U.S. Pat. No. 6,015,773 discloses a process of producing particles comprising the steps of: (a) metering a coating substance into a flow restrictor; (b) injecting a gas stream through the flow restrictor concurrently to the flow of the coating substance of step (a), in order to create a zone of turbulence at the outlet of the flow restrictor, thereby atomizing the coating substance; (c) adding a core substance to the zone of turbulence to mix the core substance with the atomized coating substance. The mixing at the zone of turbulence coats the core substance with the coating substance to from a particle having a diameter in the range of 0.5 to 50 µm. The coating substance is selected from the group consisting of wood rosin, rosin derivatives, waxes, fatty derivatives, sterols and long-chain sterol esters.

U.S. Pat. No. 6,087,003 also discloses a microparticle and a producing method thereof by using an atomized flow restrictor. The microparticle has an active substance as a central core made of a liquid, gaseous or solid particle of regular or irregular shape. The method comprises entrapping the active substance in a coating substance that is conformationally distributed on the active substance and has a thickness ranging from the thickness of a monomolecular layer to about 100 µm. However, the drawbacks of this method are the difficulty in controlling the operating conditions, such as temperature, and the complexity of executing the steps. Furthermore, the process performed in the high temperature condition is taken too long.

In the conventional methods of producing particles described above, it is very difficult to control the operating conditions, and also complicated to execute the steps. Moreover, the production yield is low, the production time is excessive, and the resultant cost is too high. Further, the conventional techniques described above use organic solvents and are performed under high temperature conditions, so that the embedded materials cannot be bacteria, vaccines or proteins, etc. The organic solvents and high temperature conditions would damage these biological materials, especially the physiologically active substances.

### SUMMARY OF THE INVENTION

It is therefore an object of the invention to provide a particle with embedded chemical substances, and especially a particle with embedded physiologically active substances. The raw materials for producing the particles with embedded chemical substances are inexpensive, and the process for producing the particles is effective. Also, the particle quality and appearance are uniform and superior to those produced by conventional techniques.

The invention achieves the above-identified objective by providing a particle that comprises at least an embedded substance and a base material. The embedded substances, embedded in base materials, is ranged from about 0.1 to 70 % by weight, based on the total weight of the particle. The base material is ranged from about 30 to 99.9 % by weight, based on the total weight of the particle. In the present invention, the base material is selected from the group consisting of hydrogenated plant oil, hydrogenated animal oil, C8-C64 saturated fatty acid and the derivatives thereof.

Moreover, the particle of the present invention can be modified by optionally adjusting the proportion or the composition of the base material, depending on the fields of application. For example, if the specific gravity of the base materials is lower than 1 g/cm³, the particles will float on the surface of water. If the specific gravity of the base materials is higher than 1 g/cm³, the particles will sink in water.

The diameter of the particle according to the present invention is in the range of about 0.01 to 5 millimeters (mm).

To achieve the objective, the method for producing the particles in accordance with the present invention is further provided. The method comprises the steps of (a) preparing the base material, embedded substance and medium solution; (b) melting the base material; (c) mixing the base material with the embedded substance; and (d) transferring the mixed base material and embedded substance to the medium solution through a duct. The temperature of the medium solution is lower than the base material by at least 5°C.

Other objects, features, and advantages of the invention will become apparent from the following detailed description of the preferred but non-limiting embodiment. The following description is made with reference to the accompanying drawings.

### BRIEF DESCRIPTION OF THE DRAWINGS

Fig. 1 is cross-sectional view of a particle with embedded chemical substances in accordance with the present invention; and

Fig 2 is a block diagram of the method to produce a particle with embedded chemical substances in accordance with the present invention.

### DETAILED DESCRIPTION OF THE PREFERRED EMBODIMENTS

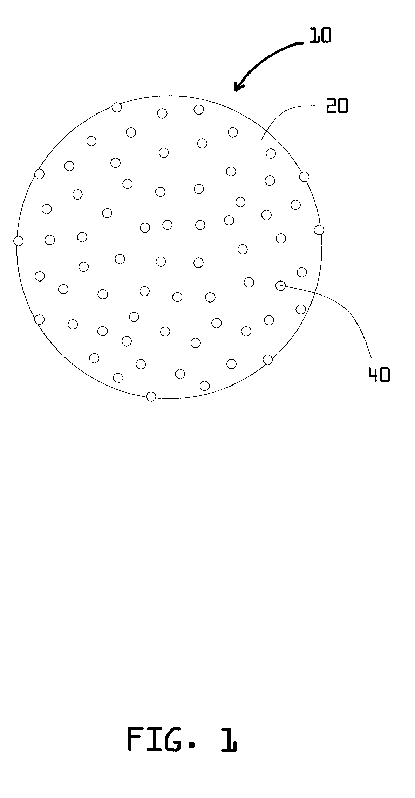

With reference to Fig. 1, the present invention relates to a particle (10) comprising a base material (20) and an embedded chemical substance (40), and especially to a particle (10) with embedded physiologically active substances.

The embedded substance (40) of the invention is in an amount of 0.1 to 70 % by weight, based on the total weight of the particle (10). The base material (20) of the invention is in an amount of 30 to 99.9 % by weight, based on the total weight of the particle (10).

The base material (20) comprises at least one member selected from the group consisting of hydrogenated plant oil, hydrogenated animal oil, C₈-C₆₄ saturated fatty acid and the derivatives thereof.

The diameter of the particle (10) in accordance with the invention is ranging from about 0.01 to 5 millimeters (mm), and preferably ranging from about 0.1 to 2 millimeters (mm).

The hydrogenated plant oil used as the base material (20) of the invention includes, for example hydrogenated coconut oil, hydrogenated palm oil, hydrogenated soybean oil and hydrogenated vegetable oil. These compounds can be used alone or in a mixture of two or more thereof.

The hydrogenated animal oil used as the base material (20) of the invention includes, for example hydrogenated butter, hydrogenated cream, hydrogenated fish oil and hydrogenated lard. These compounds can be used alone or in a mixture of two or more thereof.

The C₈-C₆₄ saturated fatty acid used as the base material (20) of the invention includes, for example caprylic acid, pelargonic acid, capric acid, undecanoic acid, dodecanoic acid (lauric acid), tridecanoic acid, myristic acid, pentadecanoic acid, palmitic acid, margaric acid, stearic acid, nonadecanoic acid, arachidic acid, heneicosanoic acid, behenic acid and the derivatives thereof. These compounds may be used alone or in a mixture of two or more thereof.

The embedded substance (40) of the invention is medicine, physiological activity substances, an inorganic compound or organic compound. Examples of embedded substance (40) include an antibiotic, hormone, vaccine, mineral or vitamin.

In one embodiment, the base material (20) is stearic acid, and the embedded substance (40) is fish protein or chitosan.

In another embodiment, the base material (20) is stearic acid or lauric acid, and the embedded substance (40) is lactobacillus.

In a further embodiment, the base material (20) is refined-hydrogenated oil, and the embedded substance (40) is beer yeast mixed with spirulina extract.

In a further embodiment, the base material (20) is stearic acid and lauric acid, and the embedded substance (40) is a vaccine that can be used for humans or animals.

In a further embodiment, the base material (20) is stearic acid, lauric acid or glycerine, and the embedded substance (40) is a vaccine that can be used for humans or animals.

In the aforementioned embodiments, there is no limitation to the mixing ratio of the base material and the embedded substance..

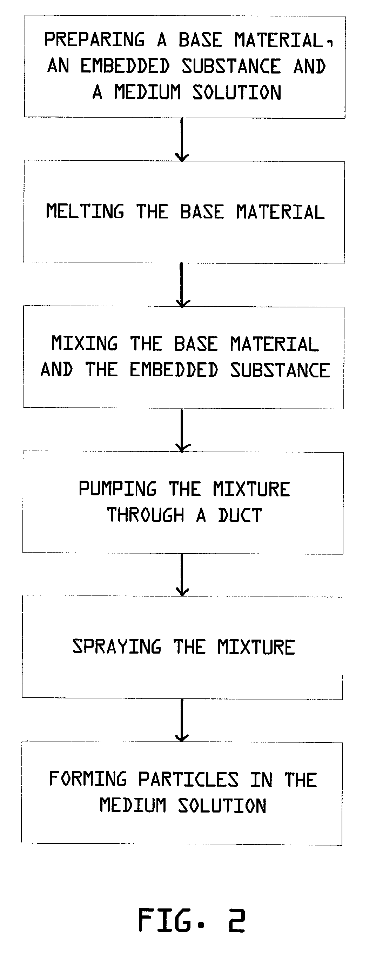

With reference to Fig. 2, the method for producing the particle (10) in accordance with the present invention comprises the steps of:
(i) preparing a base material (20), an embedded substance (40) and a medium solution;
(ii) melting the base material (20);
(iii) mixing the base material (20) and the embedded substance (40);
(iv) pumping the mixture of base material (20) and embedded substance (40) through a duct to the medium solution;
(v) breaking the mixture into micro particles, such as spraying the mixture; and
(vi) forming particles (10) in the medium solution.

It is noted that the temperature of the medium solution is maintained lower than that of the mixture of the base material and the embedded substance by at least about 5°C.

The temperature required to melt the base material (20) is in a range from about 10°C to 200°C, depending on the compositions of the base material (20). In a preferred embodiment, the temperature required to melt the base material (20) is in a range from about 40°C to 80°C.

The temperature of the medium solution is in a range from about - 10°C to 150°C. In a preferred embodiment, the temperature of the medium solution is in a range from about 20°C to 60°C.

In the pumping step (iv), the mixture of the base material (20) and the embedded substance (40) is pumped through the duct at a rate of about 0.01 liters/hour at least. In a preferred embodiment, the mixture of the base material (20) and the embedded substance (40) is pumped through the duct at a rate of about 5 to 100 liters/hour.

Furthermore, the specific gravity of the medium solution is in a range from about 0.6 to 1.5 g/cm³. The medium solution could be water, inorganic solution or organic solution. These medium solutions may be used alone or in a mixture of two or more thereof.

The principles used in the method include injecting the mixture of base material (20) and embedded substance (40) through a nozzle at a suitable temperature into the medium solution at a velocity sufficient to break the mixture into micro particles, wherein the micro particles form spheres and are cooled and solidified by the medium solution. The injection flow of the mixture the difference of specific gravity of the mixture and the medium solution make the mixture being agitated violently in the medium solution, and then being broken into a large quantity of micro particles. By means of the surface tension of the mixture (the base material (20) and embedded substance (40)) and the cohesion of the medium solution, micro particles of the mixture join to form the virtually perfect spheres suspended in the medium solution. Meanwhile, the medium solution at an adequate temperature (depending on the mixture) solidifies the spheres.

As described in the related art, the biggest flaws in coacervation are the complicated operating processes and the inability to maintain continuous production, while the biggest flaws of the atomized-flow restrictor are the high cost of equipment, the low outputs and the high rejection rate of the products. Furthermore, both methods require much more time during production. Comparing with the conventional methods, the present invention possesses the advantages including the simplification of process, no need for expensive equipment and the high production yield of particles. Table l is the comparison between the conventional methods and the method of the invention.

**Table I**

| | Method of present invention | Coacervation | Atomized-flow restrictor | Others |
|---|---|---|---|---|
| Brief introduction of method | Mix base material and embedded substance. Inject mixture into the medium solution. Produce particles. | Base material and embedded substance are two substances not dissolved with each other. Repeated Emulsification at high stirring rate, and join surfactants. Produce particles | Base material and embedded substance are powder or liquid. Suspend in a space with hot air, and combine each other to form particles after drying. | Mix base material and embedded substance. Drop into liquid containing ions, and particles are formed. |
| Use fatty acid as major carrier | Generally use fatty acid in solid state at room temperature. | Generally use fatty acid in liquid state at room temperature. | Fatty acid not frequently used as a major base material. | Fatty acid not frequently use because of the limit of surface tension of liquid. |
| The appearance and range of particles' radius | The particles are spherical. The radius and appearance of particles are uniform. | The radius of spherical particles does not vary widely. However, only small size particles are usually produced. | The particles are not spherical. The appearances of particles are not uniform, and have a wide range of particle size distribution. | The radius of spherical particles does not vary widely. However, the appearances of particles are not uniform. |
| Regulate the melting point and specific gravity of particles | Easy | Not easy | Not easy | Generally, specific gravity of particle can only be larger than water |
| The cost of equipment | Very low | Low | Very high | Low |
| The regulation conditions and steps of production | Easy | Complicated regulation conditions, and steps | Complicated regulation conditions | Easy |
| Output | Very high | High, but only operated by batch method | Low | Low |
| Disadvantage for base material | None | Emulsify at high stirring rate, and use large amounts of surfactants | High temperature of long duration | None |
| Particle production time | Short | Long | Long | Short |
| Limitations on embedded substance | None | Used in a few drugs | Used in a few foods, like coffee particles | Only used in foodstuff industry |

The fatty acid and hydrogenated animal/plant oil used as the base material (20) are very sensitive to temperature and pH value. A specific melting point and solidifying temperature of the base material (20) can be obtained by adjusting the ratio of the fatty acid and the hydrogenated level of the animal/plant oil, depending on what the embedded substance is. According to the present invention, the base material (20) embeds the embedded substances (40) , and the base material (20) has great acid stability. Therefore, the base material (20) can successfully carry most of the embedded substances (40) through the gastric acid in the human stomach.

According to the particular characteristics of the base material (20) comprising fatty acid and hydrogenated animal/plant oil, the particle (10) in accordance with the present invention can be used for the following purposes.

With regard to pharmaceutical applications, the fatty acid of the base material (20) has great acid stability, and prevents the embedded substances (40) from the moisture and air. Due to this reason, most of the medicine embedded in the base material (20) can pass through the human stomach before being released. The base material (20) of the invention also embeds the bitter taste associated with an oral medicine.

With regard to food applications, the fatty acid of the base material (20) is very stable, and protects the embedded substance (40) from the surrounding air and moisture. Use of the particle (10) helps the embedded food not only prevent damage from air and moisture, such as disintegration caused by acid and deliquescence, but also maintain the nutritional value of food. For this reason, spices and food can be embedded in the fatty acid, for example, lactobacillus embedded in the fatty acid passes through the human stomach. Furthermore, the appearance of food can be improved, and the nutritional value can be increased by releasing the food entirely inside the intestine.

With respect to aquatic and livestock applications, the fatty acid of the base material (20) is very stable and protects the embedded substance (40) from the air and moisture; for example, vaccines, antibodies and nutritional substances can be well embedded. The particles (10) possess a great property of being not dissolvable in water. Since the specific gravity of the particles (10) can be adjusted, the location of the particles (10) in an aquatic environment can be selected optionally, based on the specific purpose of the particles (10). Consequently, the particles (10) can be employed as fish fodder to accommodate fish that feed in different areas of the aquatic environment, for example, surface feeders, bottom feeders, etc.

In the livestock applications, antibodies and nutritional substances embedded in the base material (20) can be effectively protected. Also, damage to the antibodies and nutritional substances can be avoided during the forage preparation. Furthermore, animals can unwittingly ingest the particles (10) while eating the feedstuff.

With regard to other applications, the melting point of the particle (10) can be changed optionally, so that the particles (10) can be used to release carbon powders and pigment granules at different temperatures. Therefore, the particle (10) in accordance with the present invention applies to the printing business as well.

The present invention will be further illustrated by the following examples. However, the scope of the present invention should not be limited by these examples. Also, "% " in the examples represents the percentage by weight, based on the total weight of the particle, unless particularly specified.

### EXAMPLE 1

### Formulation:

| | | |
|---|---|---|
| (1) | stearic acid | 75% |
| (2) | fish protein | 20% |
| (3) | chitosan | 5% |

### Conditions:

The apparatus to produce particles includes a duct with a diameter of 7.9 mm and a spray nozzle with a diameter of 1.6 mm mounted on the end of the duct. The mixture flows at a rate of 25 liter/hr.

### Procedure:

Stearic acid and fish protein with chitosan were mixed at 70°C ± 3°C, and then the mixture was pumped through the duct to the medium solution by a positive displacement, screw type pump. Particles were produced from the mixture in the medium solution. The medium solution was maintained at 49°C ± 1°C.

### Results:

All the particles were spherical, and the diameter of the particles was in the range of 0.5 ± 0.2 millimeters. The specific gravity of the particles was greater than water. The melting point of the particle was about 55°C. The particles were produced at the rate of 25 kg per hour.

### Effect:

The particle effectively isolates the rotten smell of the fish protein and prevents air and water from damaging the fish protein, for example, disintegration caused by acid and deliquescence.

### EXAMPLE 2

### Formulation:

| | | |
|---|---|---|
| (1) | stearic acid | 63% |
| (2) | dodecanoic acid | 32% |
| (3) | lactobacillus | 5% |

### Conditions:

The apparatus to produce particles includes a duct with a diameter of 7.9 mm and a spray nozzle with a diameter of 2.7 mm mounted on the end of the duct. The mixture flowed at a rate of 38 liter/hr.

### Procedure:

Stearic acid, dodecanoic acid, and lactobacillus were mixed at 55°C ± 3°C, and then the mixture was pumped through the duct to the medium solution by a positive displacement, screw type pump. Particles were produced from the mixture in the medium solution. The medium solution was maintained at 38°C ± 1°C. Then, the particles were stored at 4°C.

### Results:

All the particles were spherical, and the diameter of the particles was in the range of 1.0 ± 0.2 millimeters. The specific gravity of the particles was less than water. The melting point of the particle was about 43°C. The particles were produced at the rate of 38 kg per hour.

### Effect:

The particles prevent lactobacillus from the damages caused by air and moisture. Also, embedded lactobacillus can resist against the gastric acid in the gastric juice.

### EXAMPLE 3

### Formulation:

| | | | |
|---|---|---|---|
| (1) | hydrogenated oil | | 90-55% |
| | (a) | stearic acid | 41 % |
| | (b) | palmitic acid | 57% |
| | (c) | myristic acid | 2% |
| (2) | beer yeast + spirulina extract | | 5-40% |
| (3) | chitosan | | 5% |

### Conditions:

The apparatus to produce particles includes a duct with a diameter of 7.9 mm and a spray nozzle with a diameter of 1.8 mm mounted on the end of the duct. The mixture flowed at a rate of 44.8 liter/hr.

### Procedure:

Hydrogenated oil, beer yeast and spirulina extract with chitosan were mixed at 70°C ± 3°C, and then the mixture was pumped through the duct to the medium solution by a positive displacement, screw type pump.

Particles were produced from the mixture in the medium solution. The medium solution was kept at 44.3°C ± 1°C. The particles were further dried at 40°C.

### Results:

All the particles were spherical, and the diameter of the particles was in the range of 0.5 ± 0.2 millimeters. The specific gravity of the particles was greater than water. The melting point of the particle was about 55°C. The particles were produced at the rate of 45 kg per hour.

### Effect:

The particles isolate the smell of beer yeast and spirulina extract, and prevent the beer yeast and spirulina extract from the damages caused by air and moisture. Also, the perfect spherical appearance adds the extra value of commercial product..

### EXAMPLE 4

### Formulation:

| | | |
|---|---|---|
| (1) | stearic acid | 63% |
| (2) | lauric acid | 32% |
| (3) | vaccine for aquatic animals | 5% |

### Conditions:

The apparatus to produce particles includes a duct with a diameter of 7.9 mm and a spray nozzle with a diameter of 2.7 mm mounted on the end of the duct. The mixture flowed at a rate of 38 liters/hr.

### Procedure:

Stearic acid, lauric acid, and a vaccine for aquatic animals were mixed at 55°C ± 3°C, and then the mixture was pumped through the duct to the medium solution by a positive displacement, screw type pump. Particles were produced from the mixture in the medium solution. The medium solution was kept at 38°C ± 1°C. The particles were stored at 4°C.

### Results:

All the particles were spherical, and the diameter of the particles was in the range of 1.0 ± 0.2 millimeters. The specific gravity of the particles was less than water. The melting point of the particle was about 43°C. The particles were produced at the rate of 38 kg per hour.

### Effect:

The damage to the embedded vaccine caused by air and water can be avoided. Also, the particles able to float on the surface of the water can immunize or treat the surface-feeding fish.

### EXAMPLE. 5

### Formulation:

| | | |
|---|---|---|
| (1) | stearic acid | 44.6% |
| (2) | lauric acid | 23.3% |
| (3) | glycerine | 28.6% |
| (4) | vaccine of aquatics | 4.5% |

### Conditions:

The apparatus to produce particles includes a duct with a diameter of 7.9 mm and a spray nozzle with a diameter of 2.7 mm mounted on the end of the duct. The mixture flowed at a rate of 38 liters/hr.

### Procedure:

Stearic acid, lauric acid, glycerine, and a vaccine for aquatic animals were mixed at 55°C ± 3°C, and then the mixture was pumped through the duct to the medium solution by a positive displacement, screw type pump. Particles were produced from the mixture in the medium solution. The medium solution was kept at 38°C ± 1°C. Then, the particles were stored at 4°C.

### Results:

All The particles were spherical, and the diameter of the particles was in the range of 1.0 ± 0.2 millimeters. The specific gravity of the particles was greater than water. The melting point of the particles was about 43°C. The particles were produced at a rate of 38 kg per hour.

### Effect:

The damage to the embedded substance caused by air and the water can be prevented. The particles will sink when thrown into water, so that the particles can be employed as the fish fodder or immunized nutrients for the bottom-feeding fish.

While the invention has been described by way of examples and in terms of the preferred embodiments, it is to be understood that the invention is not limited thereto. On the contrary, it is intended to cover various modifications and similar arrangements and procedures, and the scope of the appended claims therefore should be accorded the broadest interpretation so as to encompass all such modifications and similar arrangements and procedures.

## Claims

1. A particle, at least comprising:
a base material, from about 30 to 99.9 % by weight of the total weight of the particle, wherein the base material comprises at least one member selected from the group consisting of hydrogenated plant oil, hydrogenated animal oil, C8-C64 saturated fatty acid and the derivates thereof; and
an embedded substance, embedded in the base material, from about 0.1 to 70 % by weight of the total weight of the particle.

2. The particle as claimed in claim 1, wherein the diameter of the particle is ranging from about 0.01 to 5 millimeters.

3. The particle as claimed in claim 1, wherein the hydrogenated plant oil used as the base material is hydrogenated coconut oil, hydrogenated palm oil, hydrogenated soybean oil or hydrogenated vegetable oil.

4. The particle as claimed in claim 1, wherein the hydrogenated animal oil used as the base material is hydrogenated butter, hydrogenated cream, hydrogenated fish oil or hydrogenated lard.

5. The particle as claimed in claim 1, wherein the C8-C64 saturated fatty acid present in the base material is selected from the group consisting of caprylic acid, pelargonic acid, capric acid, undecanoic acid, lauric acid, tridecanoic acid, myristic acid, pentadecanoic acid, palmitic acid, margaric acid, stearic acid, nonadecanoic acid, arachidic acid, heneicosanoic acid, behenic acid and the derivatives thereof.

6. The particle as claimed in claim 1, wherein the embedded substance is a medicine, physiological activity material, inorganic compound or organic compound.

7. The particle as claimed in claims 1 or 6, wherein the embedded substance is an antibiotic, hormone, vaccine, mineral or vitamin.

8. The particle as claimed in claim 1, wherein the base material is stearic acid, and the embedded substance is fish protein or chitosan.

9. The particle as claimed in claim 1, wherein the base material is stearic acid or lauric acid, and the embedded substance is lactobacillus.

10. The particle as claimed in claim 1, wherein the base material is refined hydrogenated oil, and the embedded substance is beer yeast and spirulina extract.

11. The particle as claimed in claim 1, wherein the base material is stearic acid and lauric acid, and the embedded substance is a vaccine used for humans or animals.

12. The particle as claimed in claim 1, wherein the base material is stearic acid, lauric acid or glycerine, and the embedded substance is a vaccine used for humans or animals.

13. The method for preparing a particle as claimed in claim 1, comprising the steps of:
preparing a base material, an embedded substance and a medium solution;
melting the base material;
mixing the base material and the embedded substance; and
pumping the mixed base material and the embedded substance to the medium solution through a duct;
wherein the temperature of the medium solution is lower than that of the base material by at least about 5°C.

14. The method as claimed in claim 13, wherein the mixture of the base material and the embedded substance is pumped to the medium solution through the duct at a rate of at least about 0.01 liters per hour.

15. The method as claimed in claim 13, wherein the specific gravity of the medium solution is about 0.6 to 1.5 g/cm³.

16. The method as claimed in claims 13 or 15, wherein the medium solution is water, an inorganic solution or an organic solution.
